# NEW EUROPEAN PATENT SPECIFICATION

(11) **EP 1 896 056 B2**
(45) Date of publication and mention of the opposition decision: **30.05.2018**
(45) Mention of the grant of the patent: 03.11.2010
(21) Application number: 06784588.3
(22) Date of filing: 05.06.2006
(51) Int. Cl.: A61K 38/39, A61K 9/00, A61F 2/02

(54) **REINFORCED COLLAGEN SCAFFOLD**
VERSTÄRKTE KOLLAGENMATRIX
MATRICE DE COLLAGÈNE RENFORCÉE

(30) Priority: 17.06.2005 US 156092
(43) Date of publication of application: 12.03.2008
(73) Proprietor: ETHICON, INC., Somerville, NJ 08876-1515 (US)
(72) Inventor: YANG, Chunlin, Belle Mead, NJ 08502 (US); HAMMER, Joseph, Hillsborough, NJ 08844-1126 (US); WANG, Ziwei, Monrow, NJ 08831 (US)
(74) Representative: Kirsch, Susan Edith
(86) International application number: PCT/US2006/021753
(87) International publication number: WO 2006/138098

(56) References cited:
- WO-A-01/66130
- US-B1- 6 179 872
- DATABASE EMBASE [Online] ELSEVIER SCIENCE PUBLISHERS, AMSTERDAM, NL; 2002, TATEISHI T ET AL: "Biodegradable porous scaffolds for tissue engineering" XP002514038 Database accession no. EMB-2002242157 & JOURNAL OF ARTIFICIAL ORGANS 2002 JP, vol. 5, no. 2, 2002, pages 77-83, ISSN: 1434-7229
- GEIGER M. ET AL.: 'Collagen Sponges for Bone Regeneration with rhBMP-2' ADVANCED DRUG DELIVERY REVIEWS vol. 55, 2003, pages 1613 - 1629, XP003005088

## Description

### FIELD OF THE INVENTION

The present invention relates to a reinforced collagen scaffold. More specifically, it relates to a fiber-reinforced scaffold exhibiting neutralization capacity to enhance delivery of protein based bioactive agents which have a higher solubility under acidic conditions than at neutral pH.

### BACKGROUND OF THE INVENTION

Tissue engineering (TE) is defined as the application of engineering disciplines to either maintain existing tissue structures or to enable new tissue growth. This engineering approach generally includes the delivery of a tissue scaffold that serves as an architectural support onto which cells may attach, proliferate, and synthesize new tissue to repair a wound or defect. Tissue scaffolds typically have high open-celled porosity to allow cell migration throughout the scaffold and also to allow important nutrient-bearing fluids to flow through the scaffold to maintain the health of the cells.

Tissue engineering scaffolds that have been reported in the literature include meshes, woven structures, non-woven structures, knitted structures, three dimensional woven structures, sponges and foams.

The scaffolds are typically made of materials that are biocompatible. Often, they are made of biodegradable materials. Biodegradable materials readily break down into small segments when exposed to moist body tissue. The segments then either are absorbed by the body, or passed by the body. More particularly, the biodegraded segments do not elicit permanent chronic foreign body reaction, because they are absorbed by the body or passed from the body, such that no permanent trace or residual of the segment is retained by the body. Ideally, the biodegradable tissue scaffolds degrade at approximately the same rate as the body synthesizes new tissue to repair the wound or defect.

Biodegradable materials used in tissue engineering scaffolds may be either natural or synthetic. Often, polymers are used due to ease of processing and the ability to tailor their properties to match those of the native tissue in the repair site.

Tissue scaffolds formed out of collagen have also been reported. Collagen is the major protein component of the extracellular matrix of all connective tissue, including skin, tendons, bones, cartilage, blood vessels and ligaments. It is well established that collagens play critical functions in establishing and maintaining the structure of human tissues.

Collagen molecules consist of three polypeptide chains twined around one another to form triple helices. Twenty-five types of collagen have been discovered by protein purification, by cDNA and genomic DNA libraries screening, and bioinformatics. Type I collagen has been widely used as a biomaterial for biomedical applications for decades in such applications as injectable collagen formulations for soft tissue augmentation, hemostats for control of bleeding during surgery and after trauma, wound dressings and vascular graft coatings. It has also been used as a biodegradable carrier for controlled release of chemotherapy drug and antibiotics.

A broad range of tissue engineering products based on collagen scaffolds are currently under development, and some of them have already reached the market. For example, collagen gels seeded with fibroblasts have been used as the "dermal" layer of the artificial skin sold under the tradename APLIGRAFT (Sandoz AG, Basel, Switzerland), and collagen sponges have been used as an osteoconductive carrier of bone morphogenic protein-2 (BMP-2) for spine fusion and the treatment of long bone fractures.

Collagen based biomaterials have been formed into fibers, film, sheets, sponges and dispersions of fibrils. Many of these forms could be used as tissue engineering scaffolds in the repair or augmentation of body tissue. Generally, collagen is dispersed under acidic condition, in aqueous solution at pH of 2.0 and 3.2 and then the acidic collagen dispersion is formed into the final structure.

Collagen, though useful as a scaffold, lacks the mechanical integrity to support the tissue growing into the site of the defect. To address this issue, combinations of collagen and synthetic polymers have been reported. Collagen in solid forms can be combined with synthetic polymers, or collagen solutions can be applied to scaffolds formed of synthetic polymers. In the latter case, the synthetic polymer acts as a reinforcement for the collagen matrix.

To enhance the performance of a tissue engineering scaffold, growth factors that promote host cells or implanted cells to carry out their function can be added to the scaffold. Addition of growth factors provides the capacity to direct cellular responses within or around the scaffold resulting in the ability to manipulate the type of tissue that is formed.

Scaffolds that are able to retain growth factors of interest have particular advantage in tissue engineering. So, methods to bind growth factors to the scaffolds are desired to enhance the performance of the scaffold.

Many protein-based growth factors are highly soluble in acidic solutions, while marginally soluble at neutral or basic conditions. When these growth factor solutions are applied to scaffolds formed of synthetic polymers coated with acidic collagen solutions, retention of growth factors is poor.

In summary, tissue-engineering (TE) scaffolds are used to either maintain existing tissue structures or to enable new tissue growth. The scaffold serves as an architectural support onto which cells may attach, proliferate, and synthesize new tissue to repair a wound or defect. Scaffolds formed of synthetic polymers combined with acidic collagen solutions results in a reinforced collagen scaffolds with improved mechanical integrity when compared to collagen alone, while benefiting from the biological properties of the collagen. Protein-based growth factors that are highly soluble in acidic solutions, while marginally soluble at neutral or basic conditions, also can enhance the performance of reinforced collagen scaffolds, if the scaffolds are able to retain the growth factors.

Tetsuya et al. (Biodegradable porous scaffolds for tissue engineering, J. Artif. Organs 2001, 5:77-83) discloses a collagen scaffold comprising a collagen matrix interspersed within a reinforcing fibrous polymeric structure, no substance having basic pH is present. WO01/66130 discloses a collagen matrix having basic pH, no reinforcing fibrous polymer is present.

What is therefore needed is scaffolds formed of collagen matrix interspersed within synthetic polymer structures that are able to retain protein-based growth factors that are highly soluble in acidic solutions, while marginally soluble at neutral or basic conditions.

### SUMMARY OF THE INVENTION

The invention is a collagen scaffold. The scaffold comprises a collagen matrix having a basic pH. The collagen matrix is interspersed within a reinforcing polymeric fibrous structure and is obtainable by the process of the invention described herein. These scaffolds are able to retain protein-based growth factors that are highly soluble in acidic solutions, while marginally soluble at neutral or basic conditions.

The invention is also a process to prepare a reinforced basic collagen scaffold. The process comprises the steps of applying a collagen solution having a pH of 8-12 to a reinforcing polymeric fibrous structure, and drying the solution to form a collagen matrix interspersed within the reinforcing polymeric fibrous structure. The scaffolds prepared by this process can exhibit neutralization capacity for delivery of proteins that have higher solubilities under acidic condition than at neutral pH conditions.

The reinforced collagen scaffolds of the present invention are particularly useful for tissue engineering applications. Specifically, the scaffolds may find use for bone repair or soft tissue orthopedic indications. The scaffolds facilitate tissue infiltration for the repair or regeneration of diseased or damaged tissues.

In a particularly preferred embodiment, a bioactive agent, such as a protein-based growth factor which is highly soluble in acidic solutions, while marginally soluble at neutral or basic conditions, is added to the reinforced collagen scaffold to facilitate an enhanced healing response, as evidenced by speed of repair or accelerated maturation of repair tissue.

### BRIEF DESCRIPTIONS OF THE FIGURES

Figure 1 is a diagram showing the steps used to prepare reinforced basic collagen scaffolds.
Figure 2a shows a scanning electron micrograph (SEM) of a non-woven fiber reinforced basic collagen scaffold (120X).
Figure 2b shows a scanning electron micrograph (SEM) of a non-woven fiber reinforced basic collagen scaffold (650X).

### DETAILED DESCRIPTION OF THE INVENTION

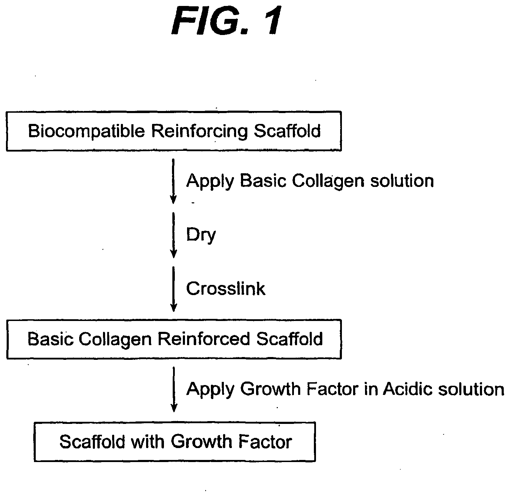

Referring to Figure 1, the process to prepare the reinforced collagen scaffolds is schematically shown. Briefly, a collagen solution having a pH of 8-12 is applied to the biocompatible reinforcing polymeric fibrous structure. The collagen solution is then dried, forming a collagen matrix within the polymeric fibrous structure. Optionally, the collagen matrix is then cross-linked. The result is a reinforced basic collagen scaffold. Finally, the protein-based growth factor that has been mixed into an acidic solution is impregnated into the reinforced collagen scaffold. The highly acid-soluble growth factor will precipitate onto the basic collagen matrix.

The biocompatible reinforcing polymeric fibrous structure useful in the present invention may be in a variety of forms including, but not limited to, meshes, woven structures, non-woven structures, knitted structures, and three dimensional woven structures.

The density and pore size distribution of the biocompatible reinforcing polymeric fibrous structure useful in the present invention is sufficient for the ingrowth of blood vessels and cells. Typical pore sizes range from about 75 to about 1,000 microns, with a preferable size range from about 100 to about 500 microns.

The polymers may either be natural or synthetic, in the preferred embodiment, the biocompatible polymer is also biodegradable.

Synthetic biodegradable biocompatible polymers are well known in the art. Such polymers are typically used to manufacture medical devices that are implanted in body tissue and absorb over time. Examples of suitable biocompatible, biodegradable polymers that could be used include polymers selected from the group consisting of aliphatic polyesters, poly(amino acids), copoly(ether-esters), polyalkylenes oxalates, polyamides, poly(iminocarbonates), polyorthoesters, polyoxaesters, polyamidoesters, polyoxaesters containing amine groups, poly(anhydrides), and blends thereof. Aliphatic polyesters include but are not limited to homopolymers and copolymers (random, block, segmented and graft) of monomers such as lactic acid, lactide(d, 1, meso and mixtures thereof), glycolic acid, glycolide, epsilon-caprolactone, trimethylene carbonate and p-dioxanone. Preferred polymers include copolymers of glycolic acid and lactic acid, such as those with a ratio of glycolic acid to lactic acid of 90/10 or 5/95.

Naturally occurring biocompatible biodegradable polymers include, but are not limited to, glycoproteins, proteoglycans, polysaccharides, glycosamineoglycan (GAG) and fragment(s) derived from these components, elastin, laminins, decrorin, fibrinogen/fibrin, fibronectins, osteopontin, tenascins, hyaluronic acid, chondroitin sulfate, heparin, ORC, carboxymethyl cellulose, and chitin

A basic pH collagen solution is applied to the biocompatible reinforcing polymeric fibrous structure. All types of collagens can be used. The basic pH collagen solution is formed by mixing collagen in a biocompatible basic buffer solution. Basic buffer solutions that could be used include phosphate, carbonate, BO₄, and tris. The preferred base is a phosphate buffer. The concentration of buffer is below about 200 milliMolar because higher concentrations of base results in basic collagen matricies that have living tissue. The pH of the solution is in the range of 8 to 12, preferably from about 10 to about 11.5. The concentration of collagen in the base solution is in the range of about 0.01 milligrams/milliliter to about 50 milligrams/milliliter, preferably from about 1 milligrams/milliliter to about 10 milligrams/milliliter.

The collagen solution is applied by any of a number of methods. Application methods include soaking, dip coating, and spray coating. Soaking will result in collagen solution penetrating deeply into, or even completely through, the biocompatible reinforcing polymeric fibrous structure. Dip coating and spray coating will result in collagen solution surface coating, or only partially penetrating into, the biocompatible reinforcing polymeric fibrous structure. Accordingly, the collagen scaffolds of this invention include those scaffolds where the collagen matrix has penetrated only a portion of the reinforcing polymeric fibrous structure.

The collagen solution is then dried. Drying methods include, but are not limited to, vacuum drying (with or without heat), drying under heat, freeze-drying or lyophilization, and drying under an inert gas flow, such as a blanket of nitrogen. The preferred drying method is lyophilization, which will create a three dimensional collagen matrix with sufficient porosity and surface area for cell infiltration and growth.

Once dried, the collagen forms a matrix interspersed within the polymeric fibrous structure, yielding a reinforced basic collagen scaffold. Advantageously, the amount of collagen loaded onto the reinforcing polymeric fibers is between about 100 micrograms to about 50 milligrams per cubic centimeter of polymeric fibrous structure. The preferred range is between about 1 to about 10 milligrams per cubic centimeter of polymeric fibrous structure. In one embodiment, the collagen matrix is cross-linked. Cross-linking of the collagen may be achieved in a number of different ways, including, but not limited to, chromium tanning, or use of a crosslinking agent, such as, but not limited to, formaldehyde, glutaraldehyde, hexamethylenediisocyanate, polyepoxy compounds, and carbodiimides. Additional crosslinking methods include acryl azide intermediate formation and physical treatments such as exposure to dry heat, ultraviolet radiation and/or gamma radiation. The preferred cross-linking technique is using formaldehyde vapor generated from a 5 to 40 percent formaldehyde solution at a temperature of 4 to 40°C for 5 minutes to 1 hour.

Next, in order to enhance the healing response through speed of repair or accelerated maturation of repair tissue, a protein-based growth factor can be mixed into an acidic solution. The protein-based growth factor is highly soluble in acidic solutions, while marginally soluble at neutral or basic conditions. One such protein is GDF5. GDF5 is a protein that functions as a growth and differentiation factor. The protein may be found in its natural state in mammals. Naturally occurring human GDF5 may be modified, purified or otherwise treated to form human recombinant RH-GDF5, as further described herein and as those skilled in the art would understand. "Recombinant Human GDF5" will be referred to generically herein as RH-GDF5. Known HR-GDF5 proteins include BMP-14, CDMP-1 and MP52.

The acidic solution of protein-based growth factor is formed by mixing the protein in a solution of water and a biocompatible acid. Acids that could be used include hydrochloric acid, acetic acid, sulfuric acid, phosphoric acid, and citric acid. The preferred acid is hydrochloric acid. The pH of the solution is in the range of about 2 to about 4, preferably from about 2.2 to about 3.5. The concentration of protein in the acid solution is in the range of about 0.001 milligrams/milliliter to about 20 milligrams/milliliter, preferably from about 0.01 milligrams/milliliter to about 1 milligrams/milliliter.

In the final step, the acidic solution of protein-based growth factor is impregnated into the reinforced basic collagen scaffold. The highly acid-soluble growth factor will precipitate onto the basic collagen. The result is a reinforced collagen scaffolds that are able to retain protein-based growth factors that are highly soluble in acidic solutions, while marginally soluble at neutral or basic conditions. In a preferred embodiment, GDF5 is precipitated onto the collagen surface as follows. GDF5 is dissolved in a 10 millimolar solution of Hydrochloric acid (HCl). GDF5 is highly soluble in this solution. However, GDF5 is relatively insoluble at neutral pH. The surface of the reinforced basic collagen scaffold possesses a basic pH with sufficient buffering capacity to neutralize the pH of the acidic GDF5 solution. Therefore, when the GDF5 is suspended in the 10 millimolar HCl and introduced to the basic collagen scaffold, the pH of the solution will approach neutral levels in the proximity of the solution/scaffold interface. The GDF5 will precipitate out of solution at this interface. The preferred range for the amount of protein on the scaffold is 0.01 to 3 micrograms per cubic centimeter of scaffold.

As noted, the collagen scaffold of this invention is preferably completely biocompatible. Furthermore, the scaffold may be entirely bioabsorbable, partially bioabsorbable, or non-bioabsorbable.

### EXAMPLES

The following examples are provided for purposes of illustration and are not intended to limit the invention in any way.

### Example 1: Preparation of reinforced basic collagen scaffold.

A dry lay non-woven needle punched reinforcing fibrous structure was made of poly(lactide-co-glycolide) (PLA/PGA) fibers. The fibers, formed of a copolymer of lactide and gycolide with lactide to glycolide weight ratio of 10:90 (or 10:90 PLA/PGA) are sold under the tradename VICRYL sutures (Ethicon, Inc., Somerville, NJ). The non-woven reinforcing structure had a nominal density of 108 milligrams per cubic centimeter, and a thickness of 2.14 millimeters. The non-woven was cut to final dimensions of about 10 centimeters X 10 centimeters.

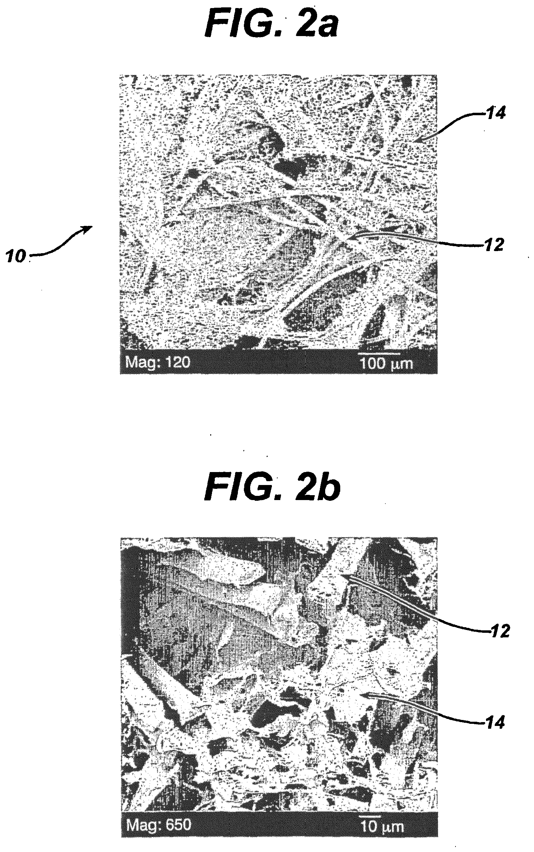

Soluble type I collagen (Kensey Nash Corporation, Exton, PA) was dissolved in 10 milliMolar sodium phosphate solution (pH 10.6) at a concentration of 5 milligrams/milliliter. The non-woven reinforcement was placed in a TEFLON-coated mold and completely soaked with the basic collagen solution. The basic collagen solution soaked non-woven structure was then lyophilized in a Durastop □P freeze-drier (FTS Systems, Stone Ridge, NY] at -25°C, 100 millitorr vacuum for 1000 minutes. The result was a reinforced basic collagen scaffold, where the amount of type 1 collagen loaded onto the reinforcing PLA/PGA fibers is 5 micrograms per cubic centimeter of polymeric fibrous structure. The collagen was stabilized by crosslinking in a closed desiccator using formaldehyde vapor generated from a 37 percent formaldehyde solution for 1 hour. Residual formaldehyde was removed by placing the scaffold in a vacuum oven set at room temperature overnight. A section of the reinforced basic collagen scaffold was examined by a scanning electron microscope, and a micrograph of a cross-section of the scaffold is shown in Figure 2. In this micrograph, the collagen matrix **14** is interspersed between the individual fibers **12** of the reinforced basic collagen scaffold **10.**

### Example 2: Retention of GDF5 in the reinforced basic collagen scaffolds.

Reinforced basic collagen scaffold, formed as in Example 1, was cut into 10-millimeter diameter discs. A second dry lay non-woven needle punched reinforcing structure was made of 10:90 PLA/PGA fibers. This structure, however, was not soaked in basic collagen solution. This structure was also cut into 10-millimeter diameter discs.

Four discs of reinforced basic collagen scaffold were transferred into two 1.8-milliliter autosampler vials (VWR, West Chester, PA), two in each vial. Four discs that were not soaked in basic collagen were also transferred into two 1.8-milliliter autosampler vials, two in each vial. Into each of the four vials, 100 micrograms (as 200 microliters of a 0.5-milligram/milliliter solution) of RH-GDF5 (Biopharm GmbH, Heidelberg, Germany), was added. After incubation for 1 hour at 4°C, the RH-GDF5 solution was squeezed out and collected by pipetting. RH-GDF5 in the collected solution was detected by RP-HPLC using a Waters HPLC system (Waters Inc, Milford, MA). The running condition for the HPLC was: Column: Vydex C18; Mobile Phase A: 0.15 percent trifluoroacetic acid (TFA) in water; Mobile Phase B: 0.15 percent TFA in acetonitrile; Flow-rate: 1 milliliter/minute; Detection wavelength: 214 nanometers.

For the reinforced basic collagen scaffolds, no GDF5 was found in the collected solution. Therefore, 100 percent of the GDF5, or 100 micrograms, was retained in the reinforced basic collagen scaffold. For the discs that were not soaked in basic collagen, however, over 90 percent of the GDF5 added to the scaffolds was found in the collected solution. Therefore, less than 10 percent of the GDF5 was retained in these discs.

### Example 3: Cell attachment on reinforced basic collagen scaffolds.

Reinforced basic collagen scaffold, formed as in Example 1, were cut into 4-millimeter diameter discs. Scaffolds were washed with phosphate buffered saline (PBS) 3 times and air-dried for 1 hour.

Eighteen of the reinforced basic collagen scaffold discs were then loaded with GDF5 by an addition of 16 microliters of a GDF5 solution made of 0.5 milligram/milliliter GDF5 in 10 milliMolar HCl to each disc. The target amount of GDF5 was 8 micrograms per scaffold. Another eighteen reinforced basic collagen scaffold discs were treated with 16 microliters of 10 milliMolar HCl. These discs served as controls.

Individual scaffolds were placed into a 6-well ultra low cluster culture plate (Corning Inc., Corning, NY) and incubated for 1 hour. Primary bovine chondrocytes were isolated from young bovine shoulder. Bovine cartilage was digested overnight with collagenase (Worthington Biochemical Corporation, Lakewood, NJ) in Dulbecco's modified Eagle's medium (Invitrogen, Carlsbad, CA). Bovine chondrocytes were seeded statically on each disc with a seeding density of about 1 x 10⁶ cells/scaffold.

Cell seeded scaffolds were incubated in 37°C humidified (100% relative humidity) air for 2 hours prior to refeeding the dish with 5 milliliters of chondrocyte growth medium with either 1 percent or 10 percent heat inactivated Fetal Bovine Serum (FBS) (HyClon, Logan, UT).

The scaffolds were cultured for 24 hours, 1 week, and 2 weeks periods. At each time period, the scaffolds were evaluated by DNA assay (CyQUANT Cell Proliferation Assay Kit; Molecular Probes, Eugene, OR) for cell number determination and by measuring the glycosamineoglycan (GAG) in the tissue. DNA content is directly translatable into the number of cells in each well, while GAG indicates matrix formation.

Table 1 shows the number of cells (in 10⁶ cells/scaffold), while Table 2 shows GAG content (in micrograms/scaffold) for all the scaffolds tested.

**Table 1: Number of cells (X 10⁻⁶) per scaffold**

| **Scaffold** | **1 Day** | **1 Week** | **2 Weeks** |
|---|---|---|---|
| Control (w/1% FBS) | 1.0±0.1 | 1.3±0.2 | 2.1±0.1 |
| Control (w/10% FBS) | 1.1±0.1 | 1.3±0.1 | 1.9±0.3 |
| GDF5 loaded (w/1% FBS) | 1.3±0.1 | 2.4±0.1 | 4.8±0.2 |
| GDF5 loaded (w/10% FBS) | 0.9±0.1 | 2.0±0.1 | 3.8±0.1 |

**Table 2: GAG content (micrograms) per scaffold**

| **Scaffold** | **1 Day** | **1 Week** | **2 Weeks** |
|---|---|---|---|
| Control (w/1% FBS) | 6.6±0.27 | 254±80 | 398±11 |
| Control (w/10% FBS) | 12.4±0.1 | 192±13 | 325±69 |
| GDF5 loaded (w/1%FBS) | 10.3±0.3 | 414±21 | 1939±42 |
| GDF5 loaded (w/10% FBS) | 12.4±0.2 | 439±73 | 2075±19 |

The tables show that the addition of RH-ODF5 to reinforced basic collagen scaffolds greatly enhances the growth of cell on the scaffolds, and cartilage specific extracellular matrix production.

## Claims

1. A method for making a collagen scaffold comprising the steps of: i) applying a basic pH collagen solution having a pH of 8 to 12 to a reinforcing polymeric fibrous structure, and ii) drying the solution to form a collagen matrix interspersed within the reinforcing polymeric fibrous structure.

2. The method of making the scaffold of Claim 1 wherein the collagen matrix is cross-linked.

3. A collagen scaffold comprising a collagen matrix having a basic pH, the collagen matrix being interspersed within a reinforcing polymeric fibrous structure, obtainable by the method of claim 1.

4. The scaffold of Claim 3 wherein the reinforcing polymeric fibrous structure is in the form of a mesh, woven structure, non- woven structure, knitted structure, three dimensional woven structure, or any combination of these.

5. The scaffold of Claim 4 wherein the reinforcing polymeric fibrous structure is composed of a polymer which is biodegradable.

6. The scaffold of Claim 5 wherein the biodegradable polymer is an aliphatic polyester.

7. The scaffold of Claim 6 wherein the aliphatic polyester is a homopolymer or copolymer of a monomer selected from the group of lactic acid, d-lactide, 1- lactide, meso-lactide, glycolic acid, glycoside, epsilon-caprolactone, trimethylene carbonate and p-dioxanone.

8. The scaffold of Claim 3 wherein the collagen matrix is composed of a Type I collagen.

9. The scaffold of Claim 8 wherein the collagen matrix is cross-linked.

10. The scaffold of Claim 3 wherein the amount of the collagen matrix loaded onto the reinforcing polymeric fibrous structure is between about 100 micrograms to about 50 milligrams per cubic centimeter of the polymeric fibrous structure.

11. The scaffold of Claim 3 wherein the amount of the collagen matrix loaded onto the reinforcing polymeric fibrous structure is between about 1 to about 10 milligrams per cubic centimeter of the polymeric fibrous structure.

12. The scaffold of Claim 3 wherein the scaffold further comprises a protein-based growth factor which is soluble in an acidic solution.

13. The scaffold of Claim 12 wherein the amount of the protein based growth factor in the collagen scaffold is between about 0.01 to about 3 micrograms per cubic centimeter of scaffold.

14. The scaffold of Claim 13 wherein the protein-based growth factor is GDF5.

## Patentansprüche

1. Verfahren zur Herstellung eines Kollagengerüstes, das die Schritte umfasst: i) Aufbringen einer Kollagenlösung mit basischem pH, die einen pH von 8 bis 12 besitzt, auf eine verstärkende polymere Faserstruktur und ii) Trocknen der Lösung, um eine Kollagenmatrix zu bilden, die in der verstärkenden polymeren Faserstruktur eingefügt ist.

2. Verfahren zur Herstellung des Gerüstes nach Anspruch 1, wobei die Kollagenmatrix vernetzt ist.

3. Kollagengerüst, das eine Kollagenmatrix mit einem basischem pH umfasst, wobei die Kollagenmatrix in einer verstärkenden polymeren Faserstruktur eingefügt ist, erhältlich durch das Verfahren nach Anspruch 1.

4. Gerüst nach Anspruch 3, wobei die verstärkende polymere Faserstruktur in der Form eines Netzes, einer gewebten Struktur, einer nicht-gewebten Struktur, einer gewirkten Struktur, einer dreidimensionalen gewebten Struktur oder irgendeiner Kombination von diesen vorliegt.

5. Gerüst nach Anspruch 4, wobei die verstärkende polymere Faserstruktur aus einem Polymer besteht, das biologisch abbaubar ist.

6. Gerüst nach Anspruch 5, wobei das biologisch abbaubare Polymer ein aliphatischer Polyester ist.

7. Gerüst nach Anspruch 6, wobei der aliphatische Polyester ein Homopolymer oder Copolymer von einem Monomer ist, das ausgewählt ist aus der Gruppe aus Milchsäure, d-Lactid, l-Lactid, meso-Lactid, Glykolsäure, Glykosid, epsilon-Caprolacton, Trimethylencarbonat und p-Dioxanon.

8. Gerüst nach Anspruch 3, wobei die Kollagenmatrix aus einem Kollagen Typ I besteht.

9. Gerüst nach Anspruch 8, wobei die Kollagenmatrix vernetzt ist.

10. Gerüst nach Anspruch 3, wobei die Menge der Kollagenmatrix, die auf die verstärkende polymere Faserstruktur geladen ist, zwischen etwa 100 Mikrogramm bis etwa 50 Milligramm pro Kubikzentimeter der polymeren Faserstruktur beträgt.

11. Gerüst nach Anspruch 3, wobei die Menge der Kollagenmatrix, die auf die verstärkende polymere Faserstruktur geladen ist, zwischen etwa 1 bis etwa 10 Milligramm pro Kubikzentimeter der polymeren Faserstruktur beträgt.

12. Gerüst nach Anspruch 3, wobei das Gerüst weiter einen Wachstumsfaktor auf Proteinbasis umfasst, der in einer sauren Lösung löslich ist.

13. Gerüst nach Anspruch 12, wobei die Menge des Wachstumsfaktors auf Proteinbasis im Kollagengerüst zwischen etwa 0,01 bis etwa 3 Mikrogramm pro Kubikzentimeter Gerüst beträgt.

14. Gerüst nach Anspruch 13, wobei der Wachstumsfaktor auf Proteinbasis GDF5 ist.

## Revendications

1. Procédé de fabrication d'un échafaudage de collagène comprenant les étapes consistant à : i) appliquer une solution de collagène à pH basique ayant un pH de 8 à 12 sur une structure fibreuse polymérique de renforcement, et ii) sécher la solution pour former une matrice de collagène intercalée à l'intérieur de la structure fibreuse polymérique de renforcement.

2. Procédé de fabrication de l'échafaudage selon la revendication 1, dans lequel la matrice de collagène est réticulée.

3. Échafaudage de collagène comprenant une matrice de collagène ayant un pH basique, la matrice de collagène étant intercalée à l'intérieur d'une structure fibreuse polymérique de renforcement, pouvant être obtenue par le procédé selon la revendication 1.

4. Échafaudage selon la revendication 3, dans lequel la structure fibreuse polymérique de renforcement est sous la forme d'une maille, d'une structure tissée, d'une structure non tissée, d'une structure tricotée, d'une structure tissée tridimensionnelle ou de n'importe quelle combinaison de celles-ci.

5. Échafaudage selon la revendication 4, dans lequel la structure fibreuse polymérique de renforcement est composée d'un polymère qui est biodégradable.

6. Échafaudage selon la revendication 5, dans lequel le polymère biodégradable est un polyester aliphatique.

7. Échafaudage selon la revendication 6, dans lequel le polyester aliphatique est un homopolymère ou copolymère d'un monomère choisi dans le groupe constitué par l'acide lactique, le d-lactide, l'l-lactide, le méso-lactide, l'acide glycolique, le glycoside, l'epsilon-caprolactone, le carbonate de triméthylène et le p-dioxanone.

8. Échafaudage selon la revendication 3, dans lequel la matrice de collagène est composée d'un collagène de type 1.

9. Échafaudage selon la revendication 8, dans lequel la matrice de collagène est réticulée.

10. Échafaudage selon la revendication 3, dans lequel la quantité de la matrice de collagène chargée sur la structure fibreuse polymérique de renforcement est entre environ 100 microgrammes et environ 50 milligrammes par centimètre cube de la structure fibreuse polymérique.

11. Échafaudage selon la revendication 3, dans lequel la quantité de la matrice de collagène chargée sur la structure fibreuse polymérique de renforcement est entre 1 et environ 10 milligrammes par centimètre cube de la structure fibreuse polymérique.

12. Échafaudage selon la revendication 3, dans lequel l'échafaudage comprend en outre un facteur de croissance à base de protéines qui est soluble dans une solution acide.

13. Échafaudage selon la revendication 12, dans lequel la quantité de facteur de croissance à base de protéines dans l'échafaudage de collagène est entre environ 0,01 et environ 3 microgrammes par centimètre cube d'échafaudage.

14. Échafaudage selon la revendication 13, dans lequel le facteur de croissance à base de protéines est GDF5.
